# EUROPEAN PATENT APPLICATION

(11) **EP 2 641 634 A1**
(43) Date of publication of application: **25.09.2013**
(21) Application number: 12160081.1
(22) Date of filing: 19.03.2012
(51) Int. Cl.: A61N 5/10, A61B 6/04, A61B 5/055

(54) **Patient support device for prone immobilization**

(71) Applicant: Université de Liège, 4031 Angleur (Liege) (BE); Centre Hospitalier Universitaire de Liège, 4000 Liège (BE)
(72) Inventor: Coucke, Philippe, 4000 Liege (BE)

(57) **Abstract**

The present invention relates to a patient immobilisation device for positioning a patient in the prone position for breast irradiation, comprising
a cephalic module for supporting the head and upper extremities of the patient,
a thoracic module for supporting the patient thorax, and shaped to allow at least one breast to extend below the thoracic module, and
a caudal module for supporting the pelvis and lower extremities of the patient,
wherein the cephalic module is optionally detachable and securable to the thoracic module; wherein the thoracic module is optionally detachable and securable to the caudal module.

## Description

### FIELD OF THE INVENTION

The present invention relates to an adjustable patient immobilization device for repeatably positioning a patient for irradiation treatment of the breast tissue. More specifically, it relates to a patient support and immobilization device for prone positioning for breast irradiation.

### BACKGROUND OF THE INVENTION

Radiation therapy plays an important role in the treatment of breast cancer. Devices for positioning patients in a precise and immobilised manner are often used in treating patients using ionizing radiation therapy. In order to control the application of radiation dose to specific localized areas of a patient; it is necessary to precisely position the patient and ensure that patient movement does not occur during the application of the therapy.

Radiation therapy treatment can be applied in either the supine position, i.e. the patient lying down on the back with the face up, as opposed to the prone position, which is face down. The current standard patient position for radiation therapy delivery of breast cancer is the supine position, especially where situation radiation of supraclavicular nodes is intended.

However, potential acute and chronic side effects are related to the treatment in the supine position. The patient may eventually develop acute and chronic pain within the radiation field. The breast itself may develop edema, dermatitis, telangiectasia, ulceration and fibrosis of the skin, with resultant undesirable cosmetic result. Yet further, overexposure of the thorax area may in some cases lead to cardiac and pulmonary complications, including ischemic heart disease, pneumonitis and pulmonary fibrosis.

Recently, prone patient immobilisation devices have been proposed, which typically consist of a board providing a cut out area for the target breast to hang free, sometimes with arm supports, forehead rest and/or lateral wells supporting the lower thorax. However, these presently known patient immobilisation supports have several shortcomings.

The adaptability to individual anatomy is limited, not allowing for adjustment for variations in arm length and/or amplitude of movement, as well as elbow and headrest position.

Yet further, these systems are usually mounted in one piece, creating a frame that is both unwieldy and heavy, and usually therefore only suitable for a single irradiation apparatus.

It therefore is considered highly desirable to have a device and a process that permits a rapid and accurate patient positioning, which offers compatibility with a number of different modern imaging and irradiating devices, and ease to handle by the treating technologists, as well as allowing to optimize planning and dose homogeneity within the target to minimize the side effects of the treatment.

Yet further, it would be highly desirable to enlarge the treatment space corresponding to the region of interest to be treated (thorax/breast) to allow more degrees of freedom for beam angulations, including non-coplanar beam orientations.

The present invention overcomes these shortcomings providing a durable patient support device for immobilizing a patient and allows precise, efficient and repeatable adjustability of the patient prone position.

### SUMMARY OF THE INVENTION

Accordingly, the present invention relates to a patient immobilisation device for positioning a patient in the prone position for breast irradiation, comprising a cephalic module for supporting the head and upper extremities of the patient, a thoracic module for supporting the patient thorax, and shaped to allow at least one breast to extend below the thoracic module, and
a caudal module for supporting the pelvis and lower extremities of the client, wherein the cephalic module is optionally detachable and securable to the thoracic module;
wherein the thoracic module is optionally detachable and securable to the caudal module; and
wherein the combined device is adaptable to be reproducibly secured to a treatment table.

The present invention is uniquely adjustable, and it is capable of repeatable positioning a patient by immobilizing the patient's head, shoulders and torso comfortably in the prone position, while giving access to the area that is to be irradiated, while minimizing the exposure of tissue to radiation. The modules are optionally secured adaptably and indexed to each other to fit the patient anatomy, and to provide a comfortable position for the patient during treatment. By comfortable position, it is herein understood that a patient can sustain a particular prone position lying on the device essentially without experiencing pain or discomfort as the result of pressure exerted on the contact points of the device for a period of time sufficient to receive a radiation treatment.

The device of the present invention is preferably adaptable to most commercially available treatment tables.

The cephalic module preferably comprises i) a base frame structure, ii) a head rest adaptably attached to the base frame, and iii) a pair of handlebars adaptably attached to the base frame, all of which can fully adjustable be mounted onto the base plate. The cephalic module immobilizes the head and arms with the head resting on the head rest, preferably executed as a forehead-chin bracket. The headrest preferably is completely adjustable to compensate for varying head sizes. Without wishing to be bound to any particular theory, it is believed that the adjustment of the headrest, more specifically the forehead support is required in order to decrease discomfort at the pressure point at the sternum.

The cephalic module further preferably comprises means to brace the arms at the elbows. A patient advantageously may gripe the handle bars; which are preferably indexed and fully adjustable to the anatomy of the patient.

The thoracic module portion supports the thorax of the patient, and disengages the breast to be treated from the remainder of the patient body. It comprises a preferably slightly curved board structure, comprising a cut-away section at the breast height. The contra lateral side of the patient thorax is supported by the slightly curved board, which is preferably padded with suitable padding material such as flexible polyurethane foam to allow a comfortable patient thorax support. The padding is preferably selected such as to minimize pressure on the patient sternum. The curvature of the board is preferably such that it completely supports the patient thorax without need for straps. This permits the patient to relax, improving reproducibility throughout the simulation and treatment process.

The cut-away section is shaped such as to allow the breast tissue to be treated to hang free through the cut-away section.

The caudal module finally passively supports the pelvis and lower extremities of the patient. It preferably provides a gradual slope that stabilizes the patient against rolling from side to side. Additional supports may preferably be present at for the patient knees and ankles to provide further comfort at articulations.

When a high energy beam is used for irradiation of the tumour, it is critical that the beam destroys the tumour but not the surrounding healthy tissue. In order to accomplish this objective with acceptable precision, it is critical that the breast tissue is maintained in a precise and fixed position with no possibility of movement. Reproducible immobilization is essential to a tighter and more conformed treatment field. By precisely positioning and repositioning a patient, a high-energy beam can be repeatably applied to a tumour. This allows for a higher dose of radiation to the gross tumour volume without affecting healthy tissue.

Applicants found surprisingly that this may be achieved by inclining or pivoting the patient according to the bodies' centre axis in order to further enhance the treatment positioning, preferably in a reproducible and accurate positioning.

Accordingly, the device is advantageously indexed pivotably along the anteroposterior patient axis. The anteroposterior patient axis is the central axis running from head to legs, also referred to as craniocaudal. The patient support device of the present invention provides a head, shoulder and torso support and immobilization device that is adaptable to most commercially available treatment tables, is easily adjustable and provides efficient repeatability while allowing to tilt the patient along the anterior-posterior axis to a certain degree, to further direct the radiation selectively.

Preferably, the subject device is executed at least in the area of irradiation, i.e. areas that are adjacent to the radiation source in a radiolucent material. Radiolucency is highly desirable, since in radiation therapy, metallic parts may case elastic and inelastic radiation scattering as well as fluorescence which can expose the patient to unnecessary radiation.

While the use of metals can cause unwanted radiation exposure, it may also reduce the desired radiation dose that reaches the target area due to their high radiation absorption compared to polymer and carbon fibre composites preferentially employed in the device according to the present invention.

Accordingly, the device is preferably executed at least in part in a polymeric composite material such as carbon fibre composite material with thermosetting resins, such as epoxy resins and/or polyesters resins. More preferably, the modules of the subject immobilization device may be essentially entirely constructed of non-metallic components to provide radiolucency.

### BRIEF DESCRIPTION OF THE DRAWINGS

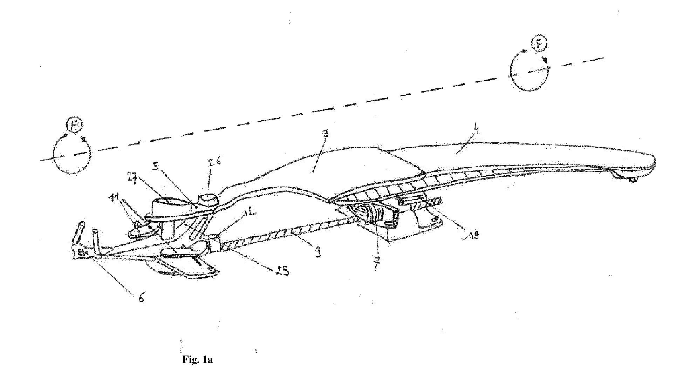

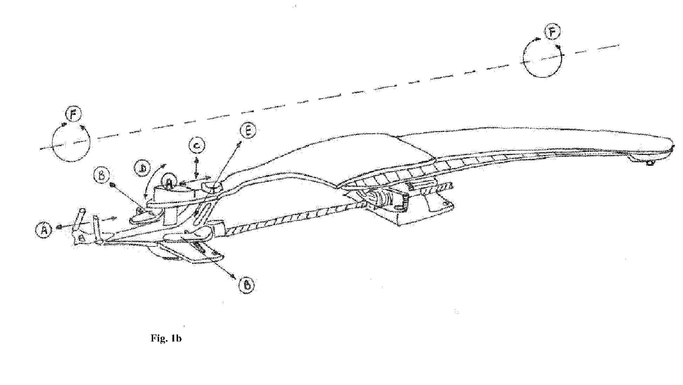

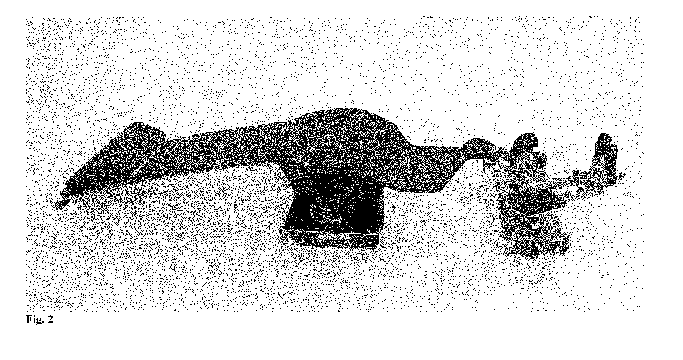

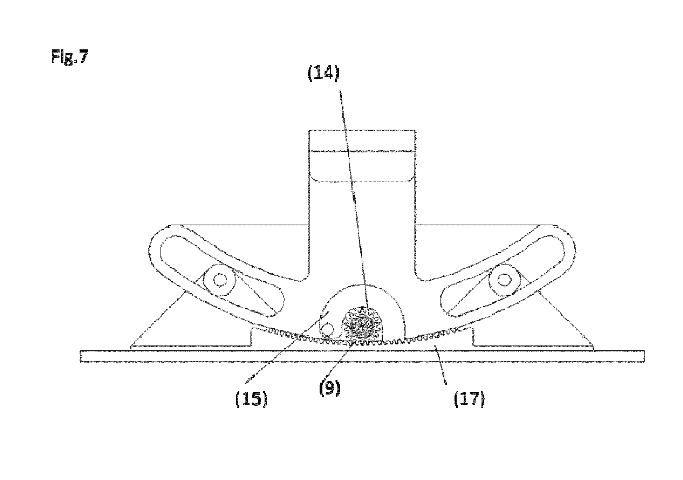

It is understood that the dimensions may vary from that shown in the drawings and the drawings are presented for illustrative purposes only. The precise shapes and dimensions of the invention can be changed without departing from the object of the present invention. Furthermore, the arrangement and specific design may change without departing from the scope of the invention. Table 1 shows the terms and relevant reference numbers employed in the figures. Table 2 refers to moving axes for an ergonomic position of the patient and an extended treatment scope. Figures 1 to 9 are illustrations of one or more preferred embodiments of the present invention. Figure 1a shows a three-dimensional depiction of the device. Figure 1b shows the same device with moving axes for an ergonomic position of the patient. Figures 2 to 6 are three-dimensional depictions of preferred embodiments of the modules and/or devices. Figures 7 to 9 are cross-sectional depictions of a preferred synchronised pivoting system.

**Table 1: List of terms and reference numbers**

| Reference Number | Term |
|---|---|
| 1 | Device |
| 2 | cephalic module |
| 3 | thoracic module |
| 4 | caudal module |
| 5 | head rest |
| 6 | Hand rest |
| 7 | base |
| 8 | primary pivoting drive/lock |
| 9 | synchronisation axis |
| 10 | secondary pivoting drive/lock |
| 11 | elbow rest |
| 12 | fixation means for treatment table 16 |
| 13 | alignment bar |
| 14 | gear |
| 15 | toothed lever |
| 16 | treatment table |
| 17 | curved rack |
| 18 | hinged lever |
| 19 | spring loaded operator grip |
| 20 | cam |
| 21 | slit |
| 22 | locking element |
| 23 | locking element |
| 24 | Slot element |
| 25 | Arm support |
| 26 | Chin support |
| 27 | Forehead support |

**Table 2: list of moving axes for comfortable position of the patient**

| Moving axes for ergonomic assemblies | | | |
|---|---|---|---|
| Ergonomic assembly | Device | Axe of moving the device | Axe of Interconnection |
| All the modules | All | F | F |
| Arms support | Hands support | A | E |
| | Elbow support | B | |
| | Arms support | E | |
| Head support | Chin | A/D | C/D |
| | Forehead | A/D | |

### DETAILED DESCRIPTION OF THE INVENTION

The device according to the invention is preferably adaptable to be reproducibly secured to a treatment table.

In Figure 1, the patient immobilisation device preferably comprises different interconnected modules arranged to rotate around a longitudinal axe or the anteroposterior patient axis F. The device is also arranged to provide a comfortable and an ergonomic position for the patient during treatment. Preferably, the modules are secured adaptably and indexed to each other to fit the patient anatomy.

The device is advantageously indexed pivotably along the anteroposterior patient axis F. The device preferably further comprises a co-rotation system for controlling of the pivoting motion of at least the cephalic and thoracic module. The patient, once placed on the device, can advantageously be inclined in an angle by means of a subsystem that acts on the whole of the patient immobilisation device. Applicants found that this leads to further distancing of the treated breast from the rest of the body, which was in particular found useful for patients with pendulous breasts.

In order to clear the treatment area as much as possible, the element bridging this area preferably has a reduced, cut-out section. As a consequence this element and its mechanical interface to the adjacent structure may be deformed under load during the inclination movement.

Therefore, to ensure proper alignment, the device advantageously comprises a synchronization system that controls the inclination of the patient immobilisation device when it is moved, as well as keeping the whole system stable and well aligned in static position. This concept is referred herein to as the "co-rotation system", and preferably comprises a number of elements. The co-rotation system preferably comprises a) at least a primary lock and at least a secondary lock, and b) at least a synchronizing element that controls and coordinates the movement of the primary and the secondary lock. The primary lock preferably has a human interface allowing an operator to change the inclination of the patient manually. The secondary lock is preferably moved by the movement of the primary lock through the synchronizing element, advantageously in the form of a synchronising axis.

The anteroposterior patient axis F may advantageously run through the patient directly, or preferably may be off-set to a parallel position below the patient. The latter preferably permits to place the pivoting mechanism below the patient, and at a position that conveniently does not interfere with the radiation or scanning equipment. In either case, the device permits to place the patient at a position at an angle with respect to the prone position along the actual anteroposterior patient axis F.

The device further preferably comprises a mechanical support structure guiding the pivoting movement of at least the cephalic and thoracic module.

Locking systems referred to as "primary and secondary locks" herein, as well as one or more elements referred to as the "synchronizing axis" that inter-connect the different lock systems and mechanically support structures that guide the inclination movement of the immobilisation device. While the primary lock preferably may have a human interface allowing an operator to change the inclination of the patient manually, the secondary locks preferably cannot be controlled directly by the operator. The locking system preferably holds the neutral (0°) and inclined positions in a safe and mechanically secured way. In the treatment area, the synchronizing axis may be removed to enhance imaging/treatment performance. When the synchronizing axis has been removed the locks are engaged and cannot be released hence in order to move the system the synchronizing axis must be in place.

The device preferably has a capacity to angle the entire board from 0° to 15°, more preferably from 3° to 9°. The pivoting action is preferably indexed in increments of 5°, more preferably in increments of 4°, and most preferably in increments of 3°. The angling or pivoting system will preferably be completely incorporated in the system to avoid unnecessary supplementary mechanical tools and reduce the weight.

To optimise the patient positioning in an ergonomic and repetitive way, the device further comprise a head rest (5), a hand rest (6), an elbow rest (11) and an arm rest(25) supports that are arranged to move independantly from each other along their respective axe (A to E). As illustrated in Figure 1b, the hand rest support (6) is arranged to move along axe A ; the elbow rest support (11), along axe (B) and the arm rest support, along axe E.

The headrest support comprises an ergonomic forehead support (27) and an ergonomic chin support (26). The chin support and the forehead support are arranged to move each independently along axes A and C and to rotate around axe D.

In a preferred embodiment, the hand rest support(6) and elbow rest support(11) are sliding supports attached on the arm support and the arm support can move along axe E.

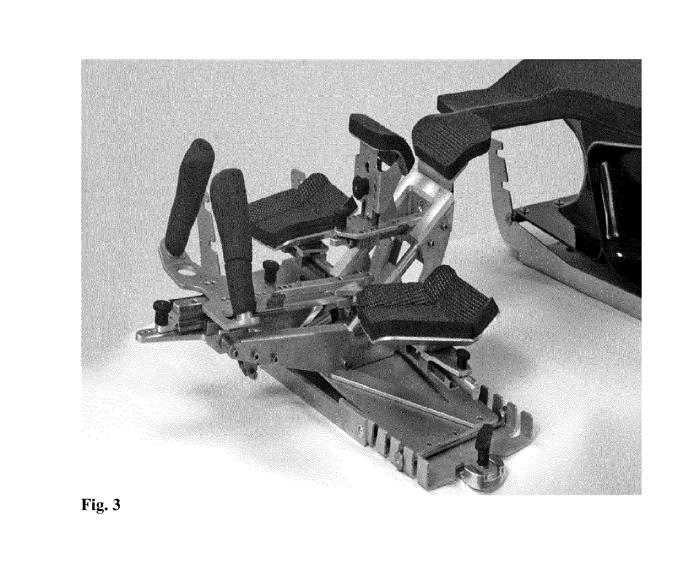

Figure 3 shows an example of cephalic module that is adaptable for receiving a head support assembly, and its fixture to a treatment table.

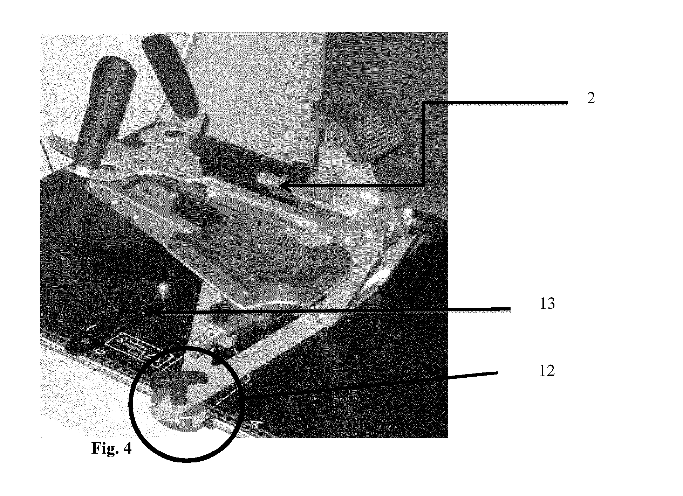

Figure 4 depicts a preferred way in which the device clamped onto the scan or treatment table through standard perforations typically present in all commercial scanning and/or radiation systems. Two alignment bars are present (at the cephalic and thoracic portion levels) to ensure identical positioning from day to day.

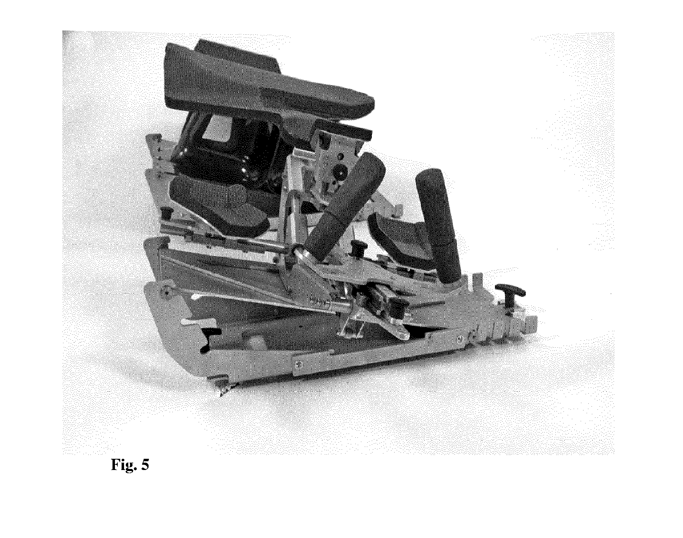

Figure 5 depicts a frontal view of an assembled device that is pivoted in a certain angle

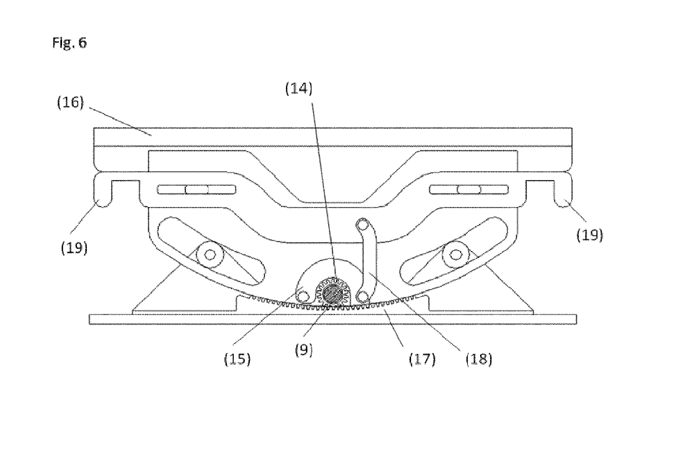

Figure 6 depicts a preferred embodiment of the device of the present invention for use with a patient in the prone position, specifically the primary lock/drive of the synchronised pivoting element. Herein, the synchronization axis (9) transfers a rotation between different supporting modules of the patient immobilisation device. The synchronization axis (9) can slide along its centre axis and has coinciding engaging positions with both primary (8) and secondary locks (10). To release the primary lock the operator moves the grip (19). When holding the grip the operator can change the inclination angle of the patient immobilisation device two-ways (CW or CCW) by pushing/pulling the patient immobilisation device's top (16).

Figure 7 depicts a preferred embodiment of the device of the present invention for use with a patient in the prone position, namely the side-on view of the secondary lock/drive linked to the first drive by synchronization axis (9). Herein, a toothed lever (15) and a curved rack (17) are linked to synchronization axis (9).

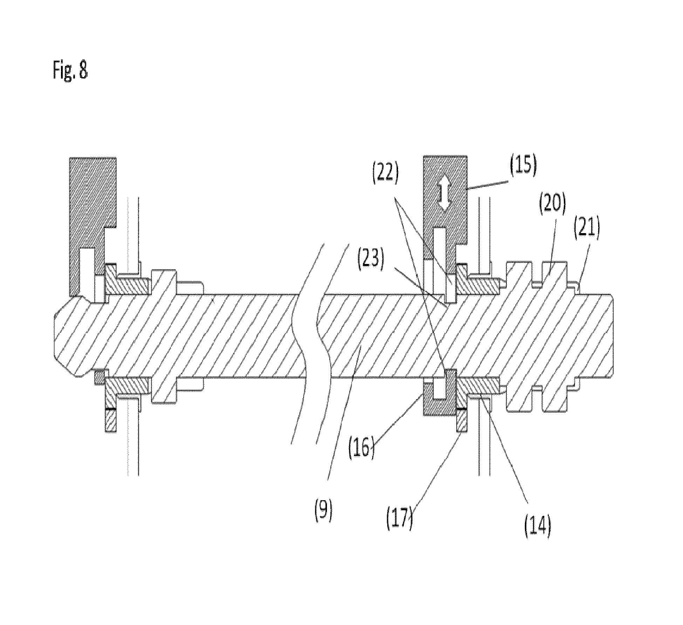

Figure 8 shows a cross-sectional view of a preferred embodiment of the primary lock. Herein, the operator activates a spring-loaded grip (19) that moves a hinged lever (18) that then lifts a toothed lever (15) which hereby is disengaged from a curved rack (17). The curved rack is part of the structural support of the patient immobilisation device and is located on a patient positioning table top (16) or similar equipment. A gear (14) transmits the rotation generated by the displacement to the synchronizing axis (9). As follows: as long as the operator holds the grip the inclination position can be changed. Once released hinged lever (18) forces toothed lever (15) back into its locking position onto the curved rack (17). The racks tooth geometry corresponds to a defined step in the inclination, for example 1 degree per tooth position. With the lock freed, i.e. operator holding grip (19), the synchronization axis (9) cannot be removed because a locking element (22) inserted into a slot (23) of the synchronization axis prevents the axis's sliding movement. Consequently when the lock is engaged, i.e grip (19) released, the geometry of the locking element (22) clears the slot of the synchronization axis allowing the operator to slide the axis and at the same time it prevents to disengage the lock. The synchronization axis, when in retracted position, remains in the primary lock, the geometry (diameter) preventing the lock to disengage by blocking element locking element (22).

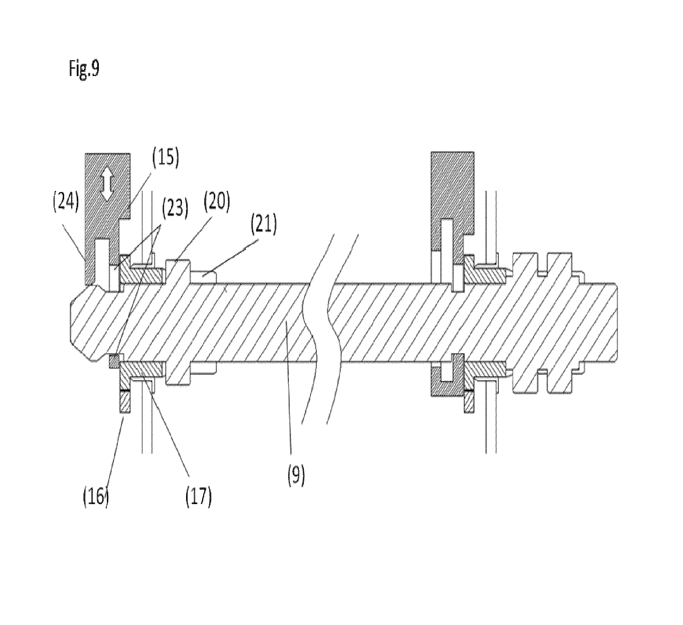

Figure 9 depicts how the secondary lock not having the human interface is controlled by means of the synchronization axis (9) position within the lock. With the axis inserted, a gear (14) and curved rack (17) can freely move and thus the inclination position can be modified by the manipulation of the operator of the primary lock, wherein rotational movement is transmitted by means of synchronization axis (9). The synchronization axis interfaces with an element (23) that is directly mounted to toothed lever (15). This element itself has a part (24) hard-mounted on it, the synchronization axis (9) geometry positioning the toothed lever, thereby clearing it from the curved rack (17). When the operator slides the synchronization axis out of the lock, the axis's geometry will move element (14) to engage the toothed lever (15) into the curved rack (17), thus blocking its position. The movement is controlled both ways by the element's (14) two-sided geometry forcing the engagement of both gear and rack. Conversely when the operator slides back the synchronization axis into the lock the gear is released. Part (24) is moves the toothed lever (15) via element (14) and the lock remains released as long as the synchronization axis (9) is in place.

The synchronization axis preferably has a number of cams (20) on along its periphery. Each set of cams is positioned on the axis corresponding to the locations of the different primary and secondary locks. The cam sets are aligned ensuring that all locks are engaged at the same time and at the same angle. When the operator moves the synchronization axis in its stored position both primary and secondary locks are disengaged in parallel and as a result all the patient immobilisation device's modules are blocked into the same inclined position. When the operator re-inserts the synchronization axis and the cams interface in slots (21) on the different gears, also these slots remain aligned geometrical disposition as described above ensures there is no geometrical conflict.

In the device according to the invention the portions of the modules that are adjacent to the irradiation source during treatment are preferably essentially radiolucent. The essentially radiolucent material may comprise a composite material, preferably carbon composite material. It more preferably does not comprise metallic parts in the portions of the modules that are adjacent to the irradiation source during treatment. The walls of at least the thoracic portion are preferably perforated to produce an open area.

The modules are preferably collapsible, to allow the device to be stowed away and/or transported in a collapsed way. The cephalic module, the thoracic module and the caudal module are adjustably connected by means that are suitable to fasten the three modules to each other. The connection is such that each module has a means for fastening the next module which is formed to receive a securing means for connecting the module to the next module.

The thoracic module preferably comprises holes, preferably symmetrical holes aligned on the side supporting the untreated breast. The triangular portion at the sternal interface is preferably adapted to the patient sternum anatomy. It is preferably padded with foam, which permits to decrease distortion of a CT planning beam.

The patient support device of the present invention can preferably be attached to most standard patient tables or couches by various attachment means. The securing means can be a removable device or it is preferably integrated into the base structure with appropriately means, such as holes and pins, so that it universally fits most common patient tables.

The patient support device of the present invention is adjustable in several ways. First, the device can be adjusted to fit most commercially available treatment tables. This adjustability provides complete flexibility in that the device of the present invention is self contained and fits most existing procedure tables. There is no need for retrofitting or additional clamping or securing means. The patient support device of the present invention adjusts on two sides and can easily be centred on a procedure table because of its two-way adjustability. The device preferably is rapidly and easily assembled to form a rigid frame. The assembled device is then preferably clamped onto the scan or treatment table through standard perforations available on all commercial systems. In a preferred embodiment, two alignment bars are present, more preferably at the cephalic and thoracic module to ensure identical positioning in subsequent treatment.

The system is preferably essentially composed from carbon fibre composites and thermosetting plastics, especially in the treatment area.

Performance of the device is directly influenced by the material used for its construction. Lighter elements are preferred over heavier elements. For example, the lighter elements in composites and polymer materials result in less elastic and inelastic radiation scattering compared to materials containing metals or alloys. In addition, fluorescence is reduced. For example, when a metal atom is impacted by radiation, it absorbs the radiation energy by ejecting an electron from its shell in the atom's electron cloud. When an electron falls back into the shell, radiation is emitted. This effect is known as fluorescence. Because the radiation can be emitted in any direction, the patient can be subj ected to an undirected dose of radiation energy.

Metals are also undesirable due to their high radiation absorption compared to plastics and carbon composite. Because of the high radiation absorption, the use of metals in the device can also reduce the therapy dose available to the patient.

All components of the present invention can be non-metallic although some metallic parts can be used if they do not disrupt the performance of the device. The individual components may be selected based on the intended use of the device, but advantageously are crafted from materials that are essentially radiolucent, such as carbon fibre epoxy resin composite. This particular feature is especially important if a highly oblique angle must be used for treating the patient where the device could come into the line of the high-energy beam.

A radiolucent device allows imaging and treatment of a patient through the patient support device. This increases the treatment flexibility by allowing an accurate attack of the cancer or tumour from all aspects and angles.

The present invention can be used in conjunction with most available tables as well as most available accessories that can be used with the patient support device.

This description and the Figures illustrate one example of the present invention and are in no way meant to be limiting. Several different specific designs are contemplated by the inventors without parting from the original scope of the present invention and would be easily recognizable by those skilled in the art. Whereas the invention has been shown and described in connection with the preferred embodiments thereof, it will be understood that many modifications, substitutions and additions can be made which are within the intended broad scope of the following claims.

## Claims

1. A patient immobilisation device (1) for positioning a patient in the prone position for breast irradiation, comprising
a cephalic module (2) for supporting the head and upper extremities of the patient,
a thoracic module (3) for supporting the patient thorax, and shaped to allow at least one breast to extend below the thoracic module, and
a caudal module (4) for supporting the pelvis and lower extremities of the patient, wherein the cephalic module is optionally detachable and securable to the thoracic module; and
wherein the thoracic module is optionally detachable and securable to the caudal module.

2. A device according to claim 1, wherein the device is adaptable to be reproducibly secured to a treatment table (16).

3. A device according to claim 1 for providing a comfortable and ergonomic position for the patient during treatment, wherein the modules are secured adaptably and indexed to each other to fit the patient anatomy.

4. A device according to any one of claims 1 to 3, wherein the device is indexed pivotably along the anteroposterior patient axis F.

5. A device according to claim 4, further comprising a co-rotation system for controlling of the pivoting motion of at least the cephalic and thoracic module.

6. A device according to claim 5, wherein the co-rotation system comprises
a) at least a primary lock (8) and at least a secondary lock (10), and
b) at least a synchronizing element (9) that controls and coordinates the movement of the primary and the secondary lock.

7. A device according to any one of claims 1 to 6, further comprising a mechanical support structure guiding the position of at least the cephalic and thoracic module.

8. A device according to any one of claims 6 to 7, wherein the primary lock has a human interface allowing an operator to change the inclination of the patient manually.

9. A device according to any one of claims 6 to 7, wherein the secondary lock (10) is moved by the movement of the primary lock (8) through the synchronizing element (9).

10. A device according to any one of the previous claims, wherein the portions of the modules that are adjacent to the irradiation source during treatment are essentially radiolucent.

11. A device according to claim 10, wherein essentially radiolucent material comprises a composite material, preferably carbon composite material.

12. A device according to any one of the previous claims, wherein the modules are collapsible, to allow the device to be stowed away and/or transported in a collapsed way.

13. A device according to any one of the previous claims, wherein the walls of at least the thoracic portion are perforated to produce an open area.

14. A process for the scanning and/or selective breast irradiation of a patient, comprising providing a cephalic module for supporting the head and upper extremities of the patient,
a thoracic module for supporting the patient thorax, and shaped to allow at least one breast to extend below the thoracic module, and
a caudal module for supporting the pelvis and lower extremities of the patient,
wherein the cephalic module is optionally detachable and securable to the thoracic module; and
wherein the thoracic module is optionally detachable and securable to the caudal module; and
subjecting at least a portion of the breast extending below the thoracic module to an ionization radiation for a suitable period of time and at a suitable radiation rate.

15. Use of the device according to any one of claims 1 to 13 for the scanning and/or selective breast irradiation of a patient.
